# EUROPEAN PATENT APPLICATION

(11) **EP 2 821 010 A1**
(43) Date of publication of application: **07.01.2015**
(21) Application number: 13754920.0
(22) Date of filing: 26.02.2013
(51) Int. Cl.: A61B 6/00, A61B 6/02, G06T 1/00

(54) **IMAGE PROCESSING DEVICE AND METHOD**

(30) Priority: 27.02.2012 JP 2012039592; 27.02.2012 JP 2012039593
(71) Applicant: FUJIFILM Corporation, Tokyo 106-8620 (JP)
(72) Inventor: IMAI, Yoshiro, Ashigarakami-gun Kanagawa 258-8538 (JP)
(74) Representative: Klunker . Schmitt-Nilson . Hirsch
(86) International application number: PCT/JP2013/001102
(87) International publication number: WO 2013/128891

(57) **Abstract**

[Aim] To accurately and efficiently suppress periodic patterns caused by a grid when plural radiographic images are obtained by imaging using the grid.

[Solution Means] A first frequency analysis unit (30) performs frequency analysis on a reference radiographic image (B1) of plural radiographic images obtained by tomosynthesis imaging using a grid, and obtains a frequency characteristic of a periodic pattern caused by the grid and a frequency analysis result (R0). A second frequency analysis unit (31) performs, based on the frequency analysis result, limited frequency analysis on a radiographic image or images (Bi (i=2 through n)) other than the reference radiographic image (B1), and obtains a frequency characteristic of the periodic pattern about the radiographic image or images (Bi). A suppression unit (32) performs, based on the frequency characteristic, processing for suppressing the periodic pattern in all of the radiographic images.

## Description

### BACKGROUND OF THE INVENTION

### Field of the Invention

The present invention relates to an image processing apparatus and method for performing image processing to suppress periodic patterns included in images.

### Description of the Related Art

Conventionally, various kinds of radiation detector for recording a radiographic image about a subject by irradiation with radiation that has passed through the subject were proposed, and put to practical use in medical fields and the like. As such radiation detectors, there is a radiation detector utilizing amorphous selenium, which generates charges by irradiation with radiation. In a radiography apparatus using this radiation detector, a scattered radiation removal grid (hereinafter, simply referred to as a grid) is provided between a radiation source for outputting radiation and the radiation detector. In the grid, lead or the like, which does not allow passage of radiation, and aluminum, wood or the like, which tends to allow passage of radiation, are alternately arranged with a predetermined pitch, and the scattered component of radiation is removed by this grid.

However, when a radiographic image of a subject is imaged with use of the grid, a periodic pattern caused by the grid, such as periodic stripes and moire, is generated as noise in the obtained radiographic image. Therefore, various modes for preventing deterioration of image qualities caused by the periodic pattern by calculating a frequency characteristic of the periodic pattern included in the radiographic image, and by performing processing for suppressing a frequency component of the periodic pattern on the radiographic image have been proposed. For example, Patent Document 1 proposes a technique in which plural linear regions are set in a radiographic image, and a frequency spectrum is obtained, as a frequency characteristic, by performing frequency analysis, such as Fourier transformation, on image signals of the linear regions, and a spatial frequency at which the response has a peak in this frequency spectrum is detected as the frequency characteristic of the periodic pattern.

Meanwhile, tomosynthesis imaging is known. In tomosynthesis imaging, radiography is performed by irradiating a subject with X-rays from different directions from each other by moving an X-ray tube, and a tomographic image in which a desirable slice plane is emphasized is obtained by adding plural radiographic images obtained by this radiography to observe a diseased part of the subject in more detail. Further, a technique for moving a grid during radiography so that moire appears at different positions from each other in a majority of a series of radiographic images has been proposed to make a periodic pattern caused by the grid less noticeable when tomosynthesis imaging is performed (please refer to Patent Document 1).

Further, as a technique for suppressing periodic patterns in plural radiographic images obtained by successive imaging, such as tomosynthesis imaging, a technique in which an image component caused by a grid is generated from one of the plural radiographic images, and the image component is removed from radiographic images obtained later has also been proposed (please refer to Patent Document 2) . When the technique disclosed in Patent Document 2 is used, it is not necessary to perform frequency analysis on all of the radiographic images. Therefore, even when plural radiographic images are obtained, it is possible to efficiently suppress periodic patterns caused by the grid.

### [Related Technical Documents]

### [Patent Documents]

[Patent Document 1]
   Japanese Unexamined Patent Publication No. 2009-219556
[Patent Document 2]
   Japanese Unexamined Patent Publication No. 2002-330343

### SUMMARY OF THE INVENTION

However, when plural radiographic images are obtained by using a grid, the frequency component of a periodic pattern included in each of the radiographic images slightly differs from each other. Further, a position at which the periodic pattern included in each of the radiographic images appears is slightly shifted from each other in some cases. For example, in tomosynthesis imaging, at least one of the position of an X-ray tube and the position of a detector changes at each imaging. Therefore, the frequency component of the periodic pattern included in each of the radiographic images slightly differs from each other, and the position of the periodic pattern included in each of the radiographic images is slightly shifted from each other. Meanwhile, the technique disclosed in Patent Document 2 uses only an image component of a periodic pattern generated from one of plural radiographic images to suppress periodic patterns in other radiographic images. Therefore, it is possible to reduce an operation amount. However, since the frequency characteristics of the periodic patterns in radiographic images other than the one of radiographic, images from which the image component of the periodic pattern has been generated, slightly differ from the frequency characteristic of the periodic pattern in the one of radiographic images, it is impossible to accurately suppress the periodic pattern.

In view of the foregoing circumstances, it is an object of the present invention to accurately and efficiently suppress periodic patterns caused by a grid when plural radiographic images are obtained by imaging using the grid.

A first image processing apparatus of the present invention includes a first frequency analysis means that obtains a frequency characteristic of a periodic pattern caused by a grid for removing a scattered component of radiation, as a first frequency characteristic, by performing frequency analysis on one of plural radiographic images obtained by successive imaging using the grid, and that obtains a frequency analysis result including the first frequency characteristic,
a second frequency analysis means that obtains a frequency characteristic of the periodic pattern about at least one radiographic image other than the one of the plural radiographic images, as a second frequency characteristic, by performing, based on the frequency analysis result, frequency analysis on the at least one radiographic image, and
a suppression means that suppresses, based on the first frequency characteristic, the periodic pattern in the one of the plural radiographic images, and that suppresses, based on the second frequency characteristic, the periodic pattern in the at least one radiographic image.

Here, radiographic images may be obtained by using a radiation detector, or by using a storage phosphor sheet utilizing storage phosphor. The storage phosphor stores a part of radiation energy by irradiation with radiation. After then, the storage phosphor outputs stimulated emission light corresponding to the stored radiation energy by irradiation with excitation light, such as visible light and laser light. When the storage phosphor sheet is used, radiographic image data are temporarily stored on the storage phosphor sheet by irradiating the storage phosphor sheet with radiation that has passed through a subject. Stimulated emission light is generated by irradiating this storage phosphor sheet with excitation light, and a radiographic image is obtained by performing photoelectric conversion on the stimulated emission light.

For example, the plural radiographic images may be obtained not only by the aforementioned tomosynthesis imaging but by energy subtraction imaging for performing energy subtraction processing using two radiographic images obtained by irradiating a subject with radiation having two different kinds of energy, or by long-size imaging for obtaining a long radiographic image by moving an X-ray tube and a radiation detector at the same time parallel to a subject, by motion-image imaging or the like.

Further, the pattern of the grid is not limited as long as a scattered component of radiation is removed. For example, the grid may be composed of plural plates arranged along a main direction or a sub-direction of the radiation detector. Alternatively, the grid may be composed of plural plates arranged in such a manner to be inclined to the main direction and the sub-direction of the radiation detector.

The term "periodic pattern" means noise having a periodic pattern included in a radiographic image. For example, the periodic pattern means periodic stripes, moire and the like included in a radiographic image obtained by imaging a subject by using a grid.

In the first image processing apparatus of the present invention, the second frequency analysis means may perform, based on the frequency analysis result, frequency analysis by limiting frequency analysis performed by the first frequency analysis means.

In this case, when the first and second frequency analysis means perform frequency analysis in plural directions on the radiographic images, the second frequency analysis means may perform, based on the frequency analysis result, frequency analysis by limiting directions.

Further, in this case, the second frequency analysis means may perform, based on the frequency analysis result, frequency analysis by limiting a range of frequency to be analyzed.

The second frequency analysis means may perform, based on the frequency analysis result, frequency analysis by limiting an area of the at least one radiographic image.

Meanwhile, a region with low probability of presence of a periodic pattern caused by a grid, such as a region outside of an irradiation field, a direct radiation region, a high absorption material region and a high noise region, exists in a radiographic image. In such regions, even if frequency analysis is performed, the frequency characteristic of a periodic pattern is not obtainable, or even if the frequency characteristic is obtainable, the accuracy is low. The phrase "frequency analysis by limiting an area of the radiographic image" means performing frequency analysis by excluding regions in which no periodic pattern was detectable when frequency analysis was performed by the first frequency analysis means or a frequency characteristic different from the obtained first frequency characteristic was obtained.

The term "region outside of an irradiation field" means a region in which an image of a subject is not included because the region in a radiation detector, a storage phosphor sheet or the like was not irradiated with radiation when radiography was performed by using a diaphragm for limiting an irradiation field.

The term "direct radiation region" means a high density region in a radiographic image obtained because radiation that has irradiated a subject directly reaches the radiation detector or the like without passing through the subject.

The term "high absorption material region" means a low density region included in a radiographic image obtained, for example, by using a protector for preventing a part of a subject, such as genitals, from being unnecessarily irradiated with radiation.

Here, when low-dose radiography is performed, quantum noise of radiation is noticeable in a relatively low density region. The term "high noise region" refers to a region like this region, in which quantum noise of radiation is noticeable.

In the first image processing apparatus of the present invention, successive imaging may be tomosynthesis imaging.

A first image processing method includes obtaining a frequency characteristic of a periodic pattern caused by a grid for removing a scattered component of radiation, as a first frequency characteristic, by performing frequency analysis on one of plural radiographic images obtained by successive imaging using the grid,
obtaining a frequency analysis result including the first frequency characteristic,
obtaining a frequency characteristic of the periodic pattern about at least one radiographic image other than the one of the plural radiographic images, as a second frequency characteristic, by performing, based on the frequency analysis result, frequency analysis on the at least one radiographic image,
suppressing, based on the first frequency characteristic, the periodic pattern in the one of the plural radiographic images, and
suppressing, based on the second frequency characteristic, the periodic pattern in the at least one radiographic image.

A second image processing apparatus of the present invention includes a frequency analysis means that obtains a frequency characteristic of a periodic pattern caused by a grid for removing a scattered component of radiation by performing frequency analysis on one of plural radiographic images obtained by successive imaging using the grid,
and a suppression means that suppresses, based on the frequency characteristic, the periodic pattern in the one of the plural radiographic images and at least one radiographic image other than the one of the plural radiographic images.

In the second image processing apparatus of the present invention, the suppression means may generate, based on the frequency characteristics, a filter for each of the plural radiographic images to extract a frequency component corresponding to the periodic pattern, and obtain plural radiographic images on which filtering processing has been performed by performing filtering processing on each of the plural radiographic images by using the filters, and suppress the periodic pattern in the one of the plural radiographic images and at least one radiographic image other than the one of the plural radiographic images by subtracting the plural radiographic images on which filtering processing has been performed from the plural radiographic images.

In the second image processing apparatus of the present invention, successive imaging may be tomosynthesis imaging.

A second image processing method of the present invention includes obtaining a frequency characteristic of a periodic pattern caused by a grid for removing a scattered component of radiation by performing frequency analysis on one of plural radiographic images obtained by successive imaging using the grid,
and suppressing, based on the frequency characteristic, the periodic pattern in the one of the plural radiographic images and at least one radiographic image other than the one of the plural radiographic images.

According to the first image processing apparatus and method of the present invention,a first frequency characteristic is obtained by performing frequency analysis on one of plural radiographic images obtained by successive imaging using a grid, and a frequency analysis result including the first frequency characteristic is obtained. Here, when successive imaging using the grid is performed, the position of a radiation source and the position of a radiation detector are changed or the like in each imaging. Therefore, the frequency characteristics of periodic patterns in obtained plural radiographic images slightly vary from each other. However, the direction of the grid, the kind of the grid and the like are not changed. In the present invention, frequency analysis based on the frequency analysis result is performed on at least one radiographic image other than the one of plural radiographic images. Therefore, limited frequency analysis is performable on the at least one radiographic image, compared with the case in which the first frequency characteristic was obtained. Hence, it is possible to reduce the operation amount of frequency analysis on the at least one radiographic image. Consequently, it is possible to efficiently suppress the periodic patterns included in the plural radiographic images. Further, since the periodic patterns are suppressed by calculating the first and second frequency characteristics for each radiographic image, it is possible to accurately suppress the periodic pattern that is caused by the grid and included in each radiographic image.

According to the second image processing apparatus and method of the present invention, the frequency characteristic of a periodic pattern caused by a grid is obtained by performing frequency analysis on one of plural radiographic images obtained by successive imaging using the grid, and the periodic patterns in the one of the plural radiographic images and at least one radiographic image other than the one of the plural radiographic images are suppressed based on the obtained frequency characteristic. Therefore, it is not necessary to obtain a frequency characteristic for each image. Hence, it is possible to reduce an operation amount for frequency analysis. Consequently, it is possible to efficiently suppress the periodic patterns included in the plural radiographic images. Meanwhile, when successive imaging is performed by using a grid, a position at which a periodic pattern included in each radiographic image appears is slightly shifted from each other in some cases. According to the present invention, a periodic pattern is suppressible by generating a spatial filter for extracting a periodic pattern based on the obtained frequency characteristic for each radiographic image. Therefore, it is possible to accurately suppress a periodic pattern that is caused by the grid and included in each radiographic image.

### BRIEF DESCRIPTION OF THE DRAWINGS

Figure 1 is a schematic block diagram illustrating the configuration of a radiographic image diagnosis system to which an image processing apparatus according to a first embodiment of the present invention has been applied;
Figure 2 is a diagram for explaining a tomosynthesis imaging;
Figure 3 is a schematic block diagram illustrating the configuration of a periodic pattern suppression processing unit in the first embodiment;
Figure 4 is a diagram illustrating small regions for first frequency analysis (No. 1);
Figure 5 is a diagram illustrating small regions for first frequency analysis (No. 2);
Figure 6 is a diagram illustrating an example of a frequency spectrum;
Figure 7 is a diagram for explaining a region within an irradiation field;
Figure 8 is a diagram for explaining small regions included in the region within the irradiation field;
Figure 9 is a diagram for explaining a peak frequency;
Figure 10 is a diagram illustrating a frequency component of a periodic pattern;
Figure 11 is a diagram illustrating a frequency spectrum of a processed radiographic image;
Figure 12 is a flow chart illustrating processing performed in the first embodiment;
Figure 13 is a schematic block diagram illustrating the configuration of a periodic pattern suppression processing unit in a second embodiment;
Figure 14 is a diagram for explaining periodic pattern suppression processing; and
Figure 15 is a flow chart illustrating processing performed in the second embodiment.

### DESCRIPTION OF THE PREFERRED EMBODIMENTS

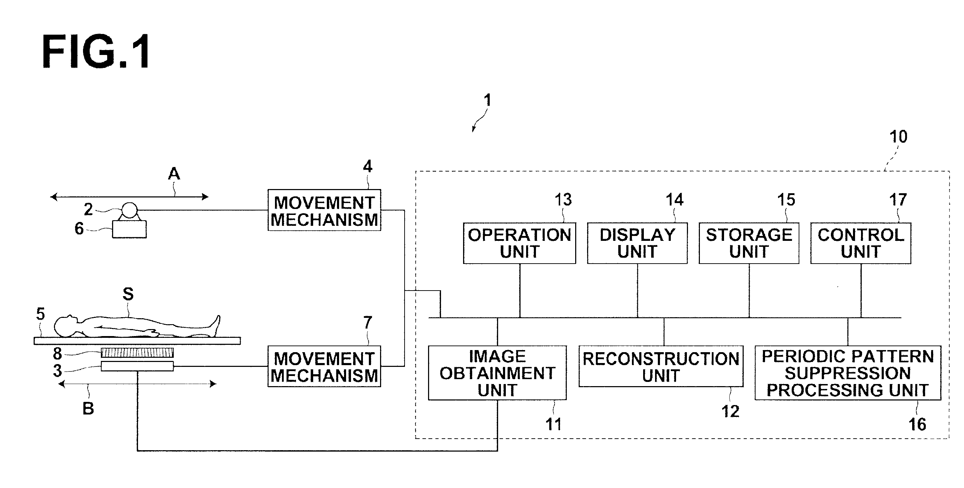

Hereinafter, embodiments of the present invention will be described with reference to drawings. Figure 1 is a schematic block diagram illustrating the configuration of a radiographic image diagnosis system to which an image processing apparatus according to a first embodiment of the present invention has been applied. As illustrated in Figure 1, this radiographic image diagnosis system 1 is used to perform tomosynthesis imaging, and includes an X-ray tube 2 and a radiation detector 3. The X-ray tube 2 is moved, along a straight line or an arc, by a movement mechanism 4, and irradiates subject S on a top plate 5 of a radiography table with X-rays at plural positions on its movement path. In the embodiment of the present invention, the X-ray tube 2 is assumed to be moved along a straight line in the direction of arrow A.

Further, a collimator (a diaphragm for limiting an irradiation field) 6 is connected to the X-ray tube 2, and an operator can set a range (an irradiation range) of X-rays irradiating subject S. Here, when the irradiation range is set by using the collimator 6, visible light instead of X-rays is output to subject S through the collimator 6. The visible light is output from an irradiation field lamp (not illustrated) provided in the collimator 6. Accordingly, the operator can set the irradiation range of X-rays by adjusting the range of visible light illuminating subject S by using the collimator 6.

The radiation detector 3 is arranged to face the X-ray tube 2 in such a manner that the top plate 5 of the radiography table, on which subject S is to be placed, is located between the radiation detector 3 and the X-ray tube 2 to detect X-rays that have passed through subject S. The radiation detector 3 stores, as an electrostatic latent image, radiographic image data of radiation that has passed through subject S. The radiation detector 3 detects, as a radiographic image, the distribution of transmittance of radiation by reading the stored electrostatic latent image. The configuration of the radiation detector 3 is not limited as long as the radiation detector 3 detects radiation and outputs the detected radiation, as image data. For example, the radiation detector 3 may be a TFT-type solid solid-state detector, or a light-readout-type solid-state detector.

Further, the radiation detector 3 is moved, along a straight line or an arc, by a movement mechanism 7 if necessary, and detects X-rays that have passed through subject S at plural positions on its movement path. In the embodiment of the present invention, the radiation detector 3 is assumed to be moved along a straight line in the direction of arrow B. In some cases, radiography is performed by moving only the X-ray tube 2 without moving the radiation detector 3.

Here, the radiographic image diagnosis system 1 is configured in such a manner that a grid 8 is detachably attachable between subject S and the radiation detector 3. Therefore, both of imaging with the grid and imaging without the grid are possible. Further, when imaging with the grid is performed, various kinds of grid (a grid ratio, a grid pattern, and the like) may be used. In the grid 8, lead, which does not allow passage of radiation, and aluminum, which allows passage of radiation, are alternately arranged, for example, with a pitch of about 4 line / mm. Further, lead is set in such a manner that the inclination of lead slightly varies according to its position so that radiation passes through aluminum and enters the radiation detector 3. The grid 8 is moved together with the radiation detector 3 by the movement mechanism 7.

Further, the radiographic image diagnosis system 1 includes an image processing apparatus 10. The image processing apparatus 10 is a computer including a high-definition liquid crystal display for displaying an image or the like, a keyboard, a mouse or the like for receiving an put by a user and a main body in which a CPU, a memory, a hard disk, a communication interface and the like are provided. The image processing apparatus 10 has a function of generating a tomographic image by reconstructing it from plural radiographic images obtained by tomosynthesis imaging, and a function of obtaining a frequency characteristic of a periodic pattern caused by a grid from a radiographic image, and further suppressing the periodic pattern. Figure 1 is a schematic block diagram illustrating the configuration of the image processing apparatus 10.

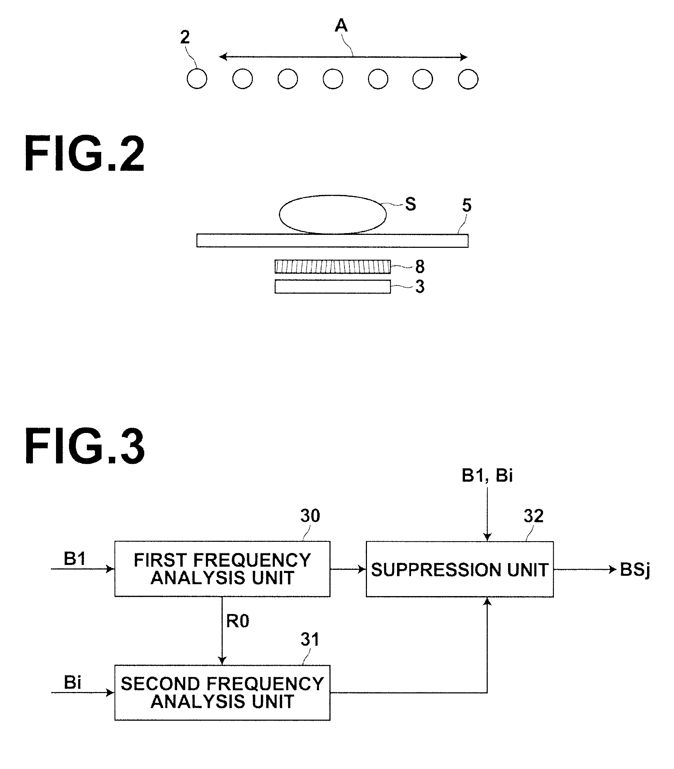

As illustrated in Figure 1, the image processing apparatus 10 includes an image obtainment unit 11 and a reconstruction unit 12. The image obtainment unit 10 moves the X-ray tube 2 along a straight line, and irradiates subject S with X-rays from plural source positions by the movement of the X-ray tube 2, and obtains plural radiographic images with the plural source positions during movement by detecting X-rays that have passed through subject S by the radiation detector 3. Specifically, as illustrated in Figure 2, radiography is performed by moving the X-ray tube 2 in the direction of arrow A, and by irradiating subject S from plural radiography directions that are different from each other. Accordingly, plural radiographic images are obtained. Here, for the purpose of explanation, the radiation detector 3 and the grid 8 are not moved in Figure 2.

The reconstruction unit 12 generates a tomographic image in which a desirable slice plane of subject S is emphasized by reconstructing the image from plural processed radiographic images on which processing for suppressing periodic patterns has been performed, as will be described later. Specifically, the reconstruction unit 12 generates a tomographic image by reconstructing the image from these processed radiographic images by using a back projection method, such as a simple back projection method and a filtered back projection method, or the like.

Further, the radiographic image diagnosis system 1 includes an operation unit 13, a display unit 14 and a storage unit 15. The operation unit 13 is composed of a key board, a mouse or a touch-panel-type input device, and receives an input of an operation to the system 1 by the user. Further, the operation unit 13 also receives an input of various kinds of information necessary to perform tomosynthesis imaging, such as conditions of radiography, and an instruction for correcting information.

The display unit 14 is a display device, such as a liquid crystal monitor. The display unit 14 displays a radiographic image obtained by the image obtainment unit 11 and a tomographic image reconstructed by the reconstruction unit 12. Further, the display unit 14 displays a message necessary for an operation, and the like. The display unit 14 may include a built-in speaker for outputting sound.

The storage unit 15 includes a hard disk for storing a radiographic image obtained by imaging, a ROM for storing various parameters for setting necessary radiography conditions to make the system 1 operate or the like, a RAM that acts as a work area, and the like.

Further, the radiographic image diagnosis system 1 includes a periodic pattern suppression processing unit 16. Figure 3 is a schematic block diagram illustrating the configuration of the periodic pattern suppression processing unit 16. As illustrated in Figure 3, the periodic pattern suppression processing unit 16 includes a first frequency analysis unit 30, a second frequency analysis unit 31 and a suppression unit 32.

The first frequency analysis unit 30 detects, as a first frequency characteristic, a frequency characteristic of a periodic pattern that is caused by the grid 8 and included in one of plural radiographic images (n images) obtained by the image obtainment unit 11 (hereinafter, referred to as reference radiographic image B1) by performing frequency analysis on reference radiographic image B1. The periodic pattern suppression processing unit 16 may sequentially perform processing on obtained radiographic images while imaging is performed. Alternatively, the periodic pattern suppression processing unit 16 may perform processing after obtainment of plural radiographic images. In the former case, a radiographic image obtained in the first imaging is used as reference radiographic image B1. In the latter case, an arbitrary radiographic image that has been set in advance, such as a radiographic image obtained in the first imaging, a radiographic image obtained in the last imaging and a radiographic image obtained in imaging at a middle position, is used as reference radiographic image B1.

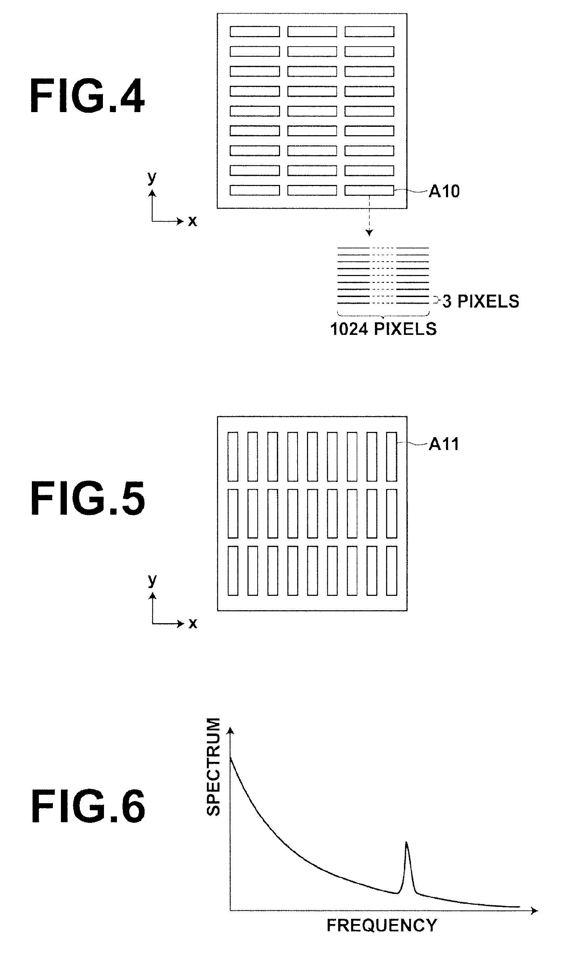

Here, the frequency analysis performed by the first frequency analysis unit 30 is referred to as first frequency analysis. Figure 4 is a diagram for explaining first frequency analysis. As illustrated in Figure 4, the first frequency analysis unit 30 sets 3x9 small regions A10 having a rectangular shape with its longitudinal side in x direction on reference radiographic image B1. Here, small region A10 includes, with intervals of three pixels, nine linear regions with a length of 1024 pixels in x direction in Figure 4. The first frequency analysis unit 30 calculates a frequency spectrum by performing Fourier transformation on image signals in each of the linear regions in small region A10. Then, the first frequency analysis unit 30 obtains an average of nine frequency spectra calculated in small region A10. Further, the first frequency analysis unit 30 calculates a frequency spectrum of reference radiographic image B1 with respect to x direction by further obtaining an average of the obtained averages of frequency spectra calculated for 3x9 small regions A10. Here, the frequency analysis using small region A10 with its longitudinal side in x direction is referred to as frequency analysis in x direction.

Further, as illustrated Figure 5, the first frequency analysis unit 30 sets plural small regions A11 with its longitudinal side in y direction on reference radiographic image B1, and calculates a frequency spectrum with respect to y direction. Here, the frequency analysis using small region A11 with its longitudinal side in y direction is referred to as frequency analysis in y direction.

Figure 6 is a diagram illustrating an example of a frequency spectrum. The frequency spectrum illustrated in Figure 6 gradually becomes lower as the spatial frequency changes from a low frequency to a high frequency, and the frequency spectrum has a peak at a certain spatial frequency. In the frequency spectrum calculated in this manner, the spatial frequency having a peak coincides with a spatial frequency of a periodic pattern caused by a grid. When the pitch of the grid 8 during radiography is x direction, a peak frequency of a periodic pattern caused by the grid appears in a frequency spectrum calculated by frequency analysis in x direction. However, no peak frequency of a periodic pattern caused by the grid appears in a frequency spectrum calculated by frequency analysis in y direction. In contrast, when the pitch of the grid 8 during radiography is y direction, a peak frequency of a periodic pattern caused by the grid appears in a frequency spectrum calculated by frequency analysis in y direction. However, no peak frequency of a periodic pattern caused by the grid appears in a frequency spectrum calculated by frequency analysis in x direction.

The first frequency analysis unit 30 obtains a frequency component that is a peak in the calculated frequency spectrum, i.e., a frequency characteristic of a periodic pattern, as a first frequency characteristic. The first frequency analysis unit 30 obtains information about a direction in which frequency analysis was performed when a peak frequency has appeared, as frequency analysis result R0, together with the first frequency characteristic. For example, when frequency analysis was performed in x direction as illustrated in Figure 4, if a peak frequency has appeared in a frequency spectrum, information of "x direction" is obtained as frequency analysis result R0. When frequency analysis was performed in y direction as illustrated in Figure 5, if a peak frequency has appeared in a frequency spectrum, information of "y direction" is obtained as frequency analysis result R0.

When the direction of the pitch of a grid is inclined with respect to a radiographic image, peaks appear in both of wave number spectra obtained by frequency analysis in x direction and by frequency analysis in y direction. When peaks have appeared in both of frequency spectra obtained by frequency analysis in x direction and by frequency analysis in y direction in this manner, the first frequency analysis unit 30 obtains information of both of "x direction" and information of "y direction", as frequency analysis result R0.

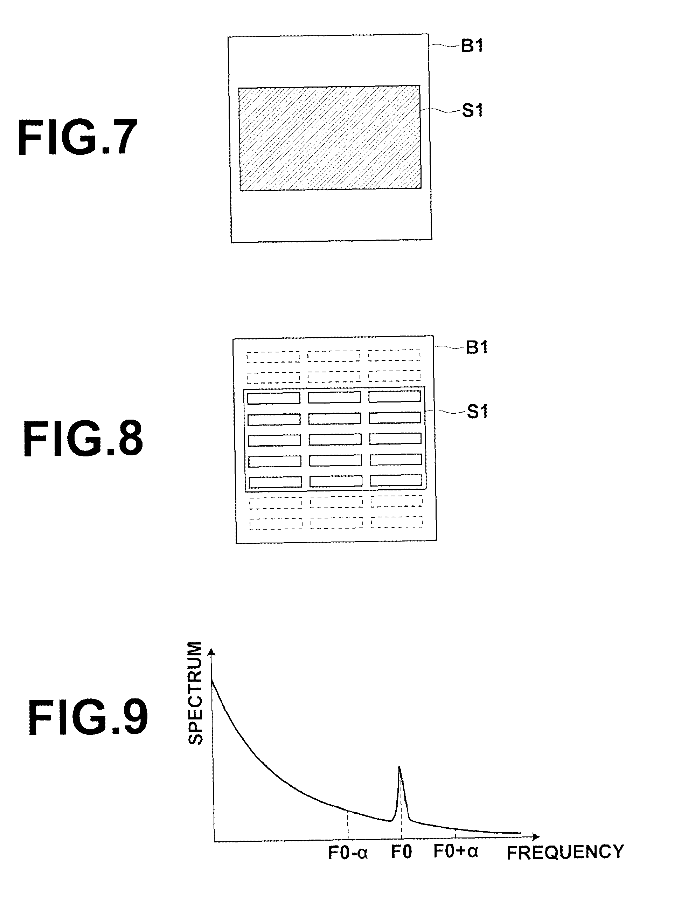

Further, since radiography is performed by limiting the irradiation range of X-rays by using the collimator 6, an image of subject S is included only in shadow part (i.e., a region within the irradiation field) S1 in reference radiographic image B1, as illustrated in Figure 7, in some cases. In such a case, even if small regions A10, A11 are set in a part of reference radiographic image B1 (i.e., a region outside of an irradiation field) other than region B1 within the irradiation field and frequency analysis is performed, no frequency spectrum is calculatable. Therefore, when small region or regions A10, All in which no spectrum was calculatable are present, the first frequency analysis unit 30 outputs information indicating the position of a small region or regions other than such a small region or regions, in other words, a small region or regions in which a spectrum was able to be calculated. For example, as illustrated in Figure 7, when reference radiographic image B1 includes region S1 within the irradiation field, information about the position of a small region or regions in region S1 within the irradiation field, which is indicated by a solid line in Figure 8, is obtained as frequency analysis result R0.

The second frequency analysis unit 31 calculates frequency characteristics of the periodic patterns caused by the grid 8 in plural other radiographic images Bi (i=2 through n) other than reference radiographic image B1 by performing, based on frequency analysis result R0 obtained by the first frequency analysis unit 30, frequency analysis on radiographic images Bi. The frequency analysis performed by the second frequency analysis unit 31 is referred to as second frequency analysis. The first frequency analysis unit 30 performs frequency analysis in each of x direction and y direction. However, the second frequency analysis unit 31 performs, based on frequency analysis result R0 of the first frequency analysis unit 30, frequency analysis only in a direction in which frequency analysis was performed when a peak frequency has appeared. For example, the direction in which frequency analysis was performed when a peak frequency has appeared is x direction, even if frequency analysis in y direction is performed, no peak frequency is detected. Therefore, the second frequency analysis unit 31 sets only small regions A10 with their longitudinal sides in x direction in radiographic images Bi, as illustrated in Figure 4, and performs frequency analysis.

When peaks have appeared in both of frequency spectra obtained by frequency analysis in x direction and by frequency analysis in y direction, the second frequency analysis unit 31 does limit directions to perform frequency analysis, but performs frequency analysis in both of x direction and y direction.

When information about the position of a small region or regions is included in frequency analysis result R0 of the first frequency analysis unit 30, the second frequency analysis unit 31 performs frequency analysis using only a small region or regions corresponding to the position of the small region or regions in the information. For example, when information about the position of small regions indicated by solid lines in Figure 8 is included in frequency analysis result R0, even if frequency analysis is performed by setting all small regions, small regions in which no frequency spectrum is calculatable are included. Therefore, the second frequency analysis unit 31 performs frequency analysis on other radiographic images Bi by setting a small region or regions only at a position or positions corresponding to the position or positions of a small region or regions included in frequency analysis result R0.

Here, when a peak frequency has been calculated, a frequency component of the periodic pattern in other radiographic image Bi should be a spatial frequency in the vicinity of the peak frequency. Therefore, after the second frequency analysis unit 31 calculates a frequency spectrum, the second frequency analysis unit 31 performs, based on information about the peak frequency included in frequency analysis result R0, processing for searching for a peak frequency only in a frequency band in the vicinity of the peak frequency. Here, when a peak frequency is F0, F0 ± α (α is a predetermined positive number) may be used as the frequency band in the vicinity of the peak frequency, as illustrated in Figure 9. It is desirable that the value of α is as small as possible to increase accuracy.

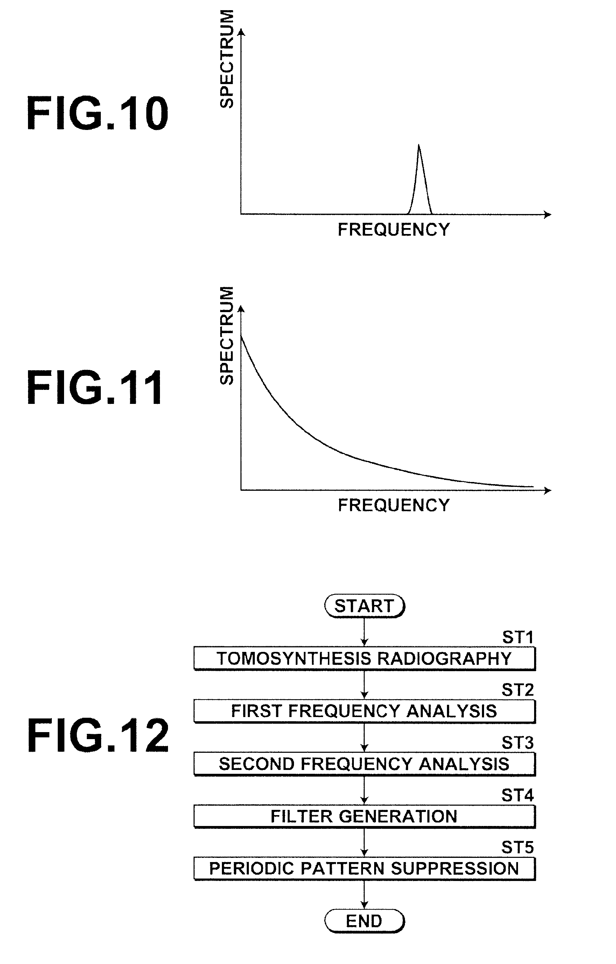

The suppressing unit 32 generates filters for reference radiographic image B1 and each of other radiographic images Bi to extract only frequency components of the periodic patterns that have been calculated about reference radiographic image B1 and other radiographic images Bi by the first and second frequency analysis units 30 and 31. Further, the suppressing unit 32 performs filtering processing on reference radiographic image B1 and each of other radiographic images Bi by using the generated filters. The frequency characteristic of a radiographic image obtained by this filtering processing includes only the frequency component corresponding to the periodic pattern, as illustrated in Figure 10. Further, the suppressing unit 32 obtains processed radiographic images BSj (j=1 through n) by subtracting the radiographic images on which filtering processing has been performed from reference radiographic image B1 and other radiographic images Bi. The frequency spectrum of processed radiographic image BSj has no peak in the frequency component of the periodic pattern caused by the grid, as illustrated in Figure 11.

Further, the radiographic image diagnosis system 1 includes a control unit 17. The control unit 17 controls each unit of the system 1 based on an instruction from the operation unit 13.

Next, processing performed in the first embodiment will be described. Figure 12 is a flow chart illustrating processing performed in the first embodiment. First, the image obtainment unit 11 obtains plural radiographic images by performing tomosynthesis imaging based on an instruction from the operation unit 13, and stores the plural radiographic images in the storage unit 15 (step ST1). Then, in the periodic pattern suppression processing unit 16, the first frequency analysis unit 30 obtains the frequency characteristic of the periodic pattern in reference radiographic image B1 of the plural radiographic images by performing first frequency analysis on reference radiographic image B1, and obtains frequency analysis result R0 (step ST2).

Then, the second frequency analysis unit 31 calculates the frequency characteristics of periodic patterns in other radiographic images Bi of the plural radiographic images other than reference radiographic image B1 by performing, based on frequency analysis result R0 output by the first frequency analysis unit 30, second frequency analysis on other radiographic images Bi (step ST3).

Then, the suppression unit 32 generates filters for reference radiographic image B1 and each of other radiographic images Bi to remove frequency components of the periodic patterns caused by the grid (step ST4). The suppression unit 32 obtains processed radiographic images BSj on which filtering processing has been performed by performing filtering processing on reference radiographic image B1 and each of other radiographic images Bi by using the generated filters (step ST5), and processing ends.

As described above, in the first embodiment, frequency analysis result R0 including the first frequency characteristic is obtained by performing frequency analysis on reference radiographic image B1, which is one of plural radiographic images obtained by successive imaging, such as tomosynthesis imaging. Here, when successive imaging using a grid is performed, the position of the X-ray tube 2 and the position of the radiation detector 3 are changed or the like in each imaging. Therefore, the frequency characteristics of periodic patterns in the radiographic images slightly vary from each other. However, the direction of the grid, the kind of the grid and the like are not changed. In the embodiment of the present invention, frequency analysis based on the frequency analysis result is performed on other radiographic image or images Bi other than reference radiographic image B1. Therefore, limited frequency analysis is performable on other radiographic image or images Bi. Hence, it is possible to reduce the operation amount of frequency analysis on other radiographic image or images Bi. Consequently, it is possible to efficiently suppress the periodic patterns included in the plural radiographic images. Further, since the periodic pattern is suppressed by calculating the first and second frequency characteristics for each radiographic image, it is possible to accurately suppress the periodic pattern that is caused by the grid and included in each radiographic image.

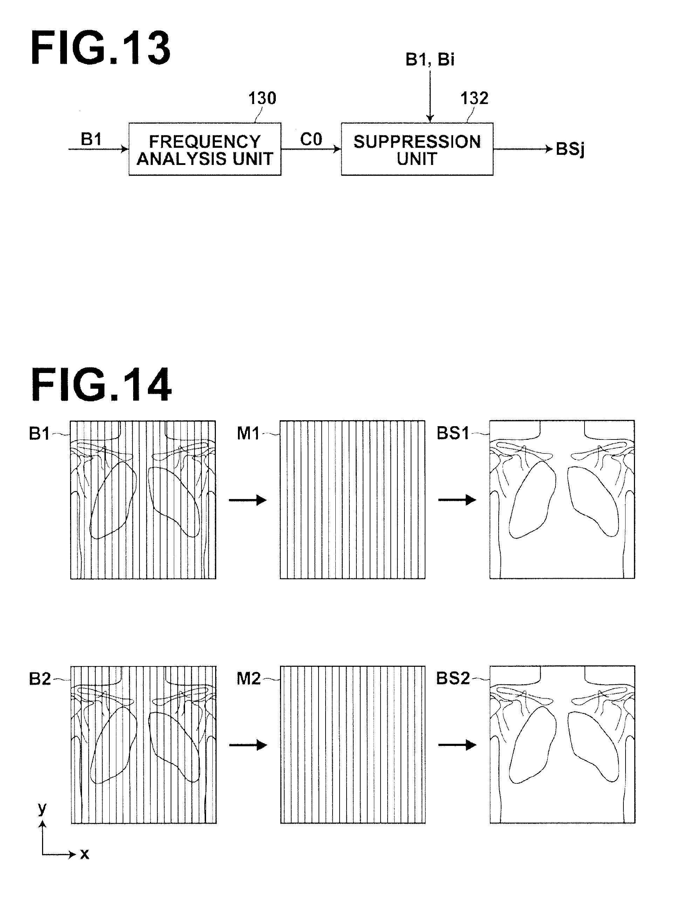

Next, a second embodiment of the present invention will be described. The second embodiment differs from the first embodiment only in processing performed by the periodic pattern suppression processing unit 16 in the first embodiment. Therefore, only the processing performed by the periodic pattern suppression processing unit 16 will be described in detail. Figure 13 is a schematic block diagram illustrating the configuration of the periodic pattern suppression processing unit 16 in the second embodiment. As illustrated in Figure 13, in the second embodiment, the periodic pattern suppression processing unit 16 includes a frequency analysis unit 130 and a suppression unit 132.

The frequency analysis unit 130 performs processing similar to the first frequency analysis performed by the first frequency analysis unit 30 in the first embodiment. The frequency analysis unit 130 obtains frequency characteristic C0 of a periodic pattern that is caused by the grid 8 and included in one (hereinafter, referred to as reference radiographic image B1) of plural radiographic images (n images) obtained by the image obtainment unit 11 by performing frequency analysis on reference radiographic image B1. The frequency analysis unit 130 obtains information about a direction in which frequency analysis was performed when a peak frequency has appeared together with frequency characteristic C0.

The suppression unit 132 generates, based on frequency characteristic C0 and the information about the direction in which frequency analysis was performed obtained by the frequency analysis unit 130, filters for extracting only frequency components corresponding to the periodic patterns for reference radiographic image B1 and each of plural other radiographic images Bi (i=2 through n) other than reference radiographic image B1. Further, the suppression unit 132 performs filtering processing on reference radiographic image B1 and each of other radiographic images Bi by using the generated filters. The frequency characteristic of the radiographic image obtained by this filtering processing includes only the frequency component corresponding to the periodic pattern, as illustrated in Figure 10. Further, the suppression unit 132 obtains processed image BSj (j=1 through n) by subtracting the radiographic images on which filtering processing has been performed from reference radiographic image B1 and other radiographic images Bi.

With reference to Figure 14, processing for suppressing a periodic pattern will be described for each of reference radiographic image B1 and radiographic image B2 of other radiographic images Bi. As illustrated in Figure 14, reference radiographic image B1 and radiographic image B2 include periodic patterns caused by the grid. However, the positions of the periodic patterns (in other words, the phases of the periodic patterns) in reference radiographic image B1 and other radiographic image B2 are shifted from each other. However, the frequency characteristic of the periodic pattern included in reference radiographic image B1 and the frequency characteristic of the periodic pattern included in other radiographic image B2 are the same. Here, since the periodic patterns are arranged in x direction in Figure 14, the information obtained by the frequency analysis unit 30 is information of x direction.

Therefore, when a filter for each of reference radiographic image B1 and radiographic image B2 is generated based on frequency characteristic C0 obtained by the frequency analysis unit 130, and filtering processing is performed for reference radiographic image B1 and radiographic image B2, radiographic images M1, M2, which include only frequency components corresponding to the periodic patterns arranged in x direction, are obtained. A periodic pattern in radiographic image M1 and a periodic pattern in radiographic image M2 appear at the same positions as the positions of the periodic patterns in reference radiographic image B1 and radiographic image B2, respectively. Therefore, processed radiographic images BS1, BS2 obtained by subtracting radiographic images M1, M2 from reference radiographic image B1 and radiographic image B2, respectively, are images from which the periodic patterns have been removed. Accordingly, the frequency spectrum of processed radiographic image BSj has no peak in the frequency component of the periodic pattern caused by the grid, as illustrated in Figure 11.

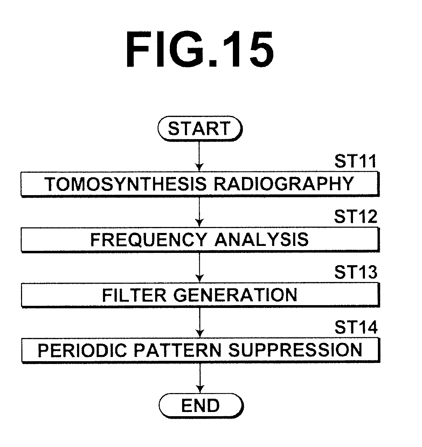

Next, processing performed in the second embodiment will be described. Figure 15 is a flow chart illustrating processing performed in the embodiment of the present invention. First, the image obtainment unit 11 obtains plural radiographic images by performing tomosynthesis imaging based on an instruction from the operation unit 13, and stores the plural radiographic images in the storage unit 15 (step ST11). Then, in the periodic pattern suppression processing unit 16, the frequency analysis unit 130 obtains frequency characteristic CO of the periodic pattern in reference radiographic image B1 by performing frequency analysis on reference radiographic image B1 of the plural radiographic images (step ST12).

Then, the suppression unit 132 generates, based on obtained frequency characteristic CO, filters for reference radiographic image B1 and each of other radiographic images Bi to remove frequency components corresponding to the periodic patterns caused by the grid (step ST13). The suppression unit 132 obtains processed radiographic images BSj by performing processing for suppressing the periodic patterns on reference radiographic image B1 and each of other radiographic images Bi by using the generated filters (step ST14), and processing ends.

As described above, in the second embodiment, frequency characteristic CO of the periodic pattern caused by the grid is obtained by performing frequency analysis on reference radiographic image B1, which is one of plural radiographic images obtained by successive imaging, such as tomosynthesis imaging. Further, the periodic patterns in reference radiographic image B1 and other radiographic image Bi are suppressed based on obtained frequency characteristic C0. Therefore, it is not necessary to obtain frequency characteristic C0 for each image. Hence, it is possible to reduce an operation amount for frequency analysis. Consequently, it is possible to efficiently suppress the periodic patterns included in the plural radiographic images. Further, a filter for extracting a periodic pattern is generated based on obtained frequency characteristic C0 for each radiographic image, and a periodic pattern is suppressible. Therefore, it is possible to accurately suppress the periodic pattern that is caused by the grid and included in each radiographic image.

In the first and second embodiments, the image processing apparatus of the present invention is applied to a radiographic image diagnosis system 1 that performs tomosynthesis imaging. The image processing apparatus of the present invention is applicable to any kind of system as long as the system obtains plural radiographic images by successive imaging. Two successive radiographic images are obtained, for example, also in energy subtraction imaging. Energy subtraction imaging is performed to perform energy subtraction processing using two radiographic images obtained by irradiating a subject with radiation having two different kinds of energy. Therefore, in the first embodiment, when the first frequency analysis unit 30 obtains a frequency analysis result by performing frequency analysis on one of two radiographic images, and the second frequency analysis unit 31 performs, based on this obtained result, frequency analysis on the other radiographic image, it is possible to reduce the operation amount of frequency analysis performed on the other radiographic image. Further, in the second embodiment, when the frequency analysis unit 130 obtains frequency characteristic C0 by performing frequency analysis on one of the two radiographic images, and performs processing for suppressing periodic patterns in the two radiographic images, it is possible to reduce the operation amount of frequency analysis performed on the other radiographic image.

Further, plural radiographic images are successively obtainable also by long-size imaging for obtaining a long radiographic image by moving an X-ray tube and a radiation detector at the same time parallel to a subject, by motion-image imaging, or the like. Therefore, similarly, also with respect to radiographic images obtained by long-size imaging or by motion-image imaging, when the second frequency analysis unit 31 performs frequency analysis based on the frequency analysis result obtained by the first frequency analysis unit 30 in the first embodiment, or when periodic patterns in plural radiographic images are suppressed based on frequency characteristic C0 obtained by the frequency analysis unit 30 in the second embodiment, it is possible to reduce the operation amount of frequency analysis.

Here, when plural radiographic images are obtained by motion-image imaging, in the case of the first embodiment, a frequency analysis result about the first frame may be used to perform frequency analysis on the frames after the first frame. Alternatively, a frequency analysis result may be obtained, for example, by performing frequency analysis on a frame in every predetermined number of frames by the first frequency analysis unit 30, and frequency analysis may be performed on the other frames in the predetermined number of frames by using this result. In the case of the second embodiment, periodic patterns in the first frame and the frames after the first frame may be suppressed based on frequency characteristic C0 obtained by performing frequency analysis on the first frame. Alternatively, frequency characteristic C0 may be obtained, for example, by performing frequency analysis on a frame in every predetermined number of frames by the frequency analysis unit 30, and periodic patterns in the predetermined number of frames may be suppressed by using this. The predetermined number may be 2. In such a case, in the first embodiment, a frequency analysis result is obtained in every two frames, and frequency analysis on the next frame is performed based on the frequency analysis result. Further, in the second embodiment, frequency characteristic C0 is obtained in every two frames, and periodic patterns in two frames are suppressed based on this.

In the first embodiment, frequency analysis is performed on all of plural other radiographic images Bi. Alternatively, frequency analysis may be performed only on at least one necessary radiographic image.

Further, in the second embodiment, processing for suppressing a periodic pattern is performed on all of plural other radiographic images Bi. Alternatively, processing for suppressing a periodic pattern may be performed only on at least one necessary radiographic image.

Further, in the first embodiment, the second frequency analysis unit 31 performs frequency analysis without using a small region or regions located outside of an irradiation field. Meanwhile, a region with low probability of presence of a periodic pattern caused by a grid, such as a direct radiation region, a high absorption material region and a high noise region, exists in a radiographic image. In such regions, even if frequency analysis is performed, the frequency characteristic of a periodic pattern is not obtainable, or even if the frequency characteristic is obtainable, the accuracy is low.

Therefore, the first frequency analysis unit 30 may detect at least one of a direct radiation region, a high absorption material region and a high noise region. Further, information about the position of small region or regions A10, A11 included in these regions may be included in the frequency analysis result. Further, the second frequency analysis unit 31 may perform frequency analysis without using the small region or regions included in the direct radiation region, the high absorption material region and the high noise region.

Further, in the first embodiment, the second frequency analysis unit 31 performs frequency analysis by limiting a direction, an area and a frequency band. Alternatively, frequency analysis may be performed by limiting at least one of them.

In the first embodiment and the second embodiment, radiographic images of subject S are obtained by using the radiation detector 3. Alternatively, radiographic images may be obtained by using a storage phosphor sheet utilizing storage phosphor. The storage phosphor stores a part of radiation energy by irradiation with radiation. After then, the storage phosphor outputs stimulated emission light corresponding to the stored radiation energy by irradiation with excitation light, such as visible light and laser light. When the storage phosphor sheet is used, radiographic image data are temporarily stored on the storage phosphor sheet by irradiating the storage phosphor sheet with radiation that has passed through a subject. Stimulated emission light is generated by irradiating this storage phosphor sheet with excitation light, and a radiographic image is obtained by performing photoelectric conversion on the stimulated emission light.

## Claims

1. An image processing apparatus comprising:
a first frequency analysis means that obtains a frequency characteristic of a periodic pattern caused by a grid for removing a scattered component of radiation, as a first frequency characteristic, by performing frequency analysis on one of a plurality of radiographic images obtained by successive imaging using the grid, and that obtains a frequency analysis result including the first frequency characteristic;
a second frequency analysis means that obtains a frequency characteristic of the periodic pattern about at least one radiographic image other than the one of the plurality of radiographic images, as a second frequency characteristic, by performing, based on the frequency analysis result, frequency analysis on the at least one radiographic image; and
a suppression means that suppresses, based on the first frequency characteristic, the periodic pattern in the one of the plurality of radiographic images, and that suppresses, based on the second frequency characteristic, the periodic pattern in the at least one radiographic image.

2. The image processing apparatus, as defined in Claim 1, wherein the second frequency analysis means performs, based on the frequency analysis result, frequency analysis by limiting frequency analysis performed by the first frequency analysis means.

3. The image processing apparatus, as defined in Claim 2, wherein when the first and second frequency analysis means perform frequency analysis in a plurality of directions on the radiographic images, the second frequency analysis means performs, based on the frequency analysis result, frequency analysis by limiting directions.

4. The image processing apparatus, as defined in Claim 2 or 3, wherein the second frequency analysis means performs, based on the frequency analysis result, frequency analysis by limiting a range of frequency to be analyzed.

5. The image processing apparatus, as defined in any one of Claims 2 through 4, wherein the second frequency analysis means performs, based on the frequency analysis result, frequency analysis by limiting an area of the at least one radiographic image.

6. The image processing apparatus, as defined in any one of Claims 1 to 5, wherein successive imaging is tomosynthesis imaging.

7. An image processing method comprising:
obtaining a frequency characteristic of a periodic pattern caused by a grid for removing a scattered component of radiation, as a first frequency characteristic, by performing frequency analysis on one of a plurality of radiographic images obtained by successive imaging using the grid;
obtaining a frequency analysis result including the first frequency characteristic;
obtaining a frequency characteristic of the periodic pattern about at least one radiographic image other than the one of the plurality of radiographic images, as a second frequency characteristic, by performing, based on the frequency analysis result, frequency analysis on the at least one radiographic image;
suppressing, based on the first frequency characteristic, the periodic pattern in the one of the plurality of radiographic images; and
suppressing, based on the second frequency characteristic, the periodic pattern in the at least one radiographic image.

8. An image processing apparatus comprising:
a frequency analysis means that obtains a frequency characteristic of a periodic pattern caused by a grid for removing a scattered component of radiation by performing frequency analysis on one of a plurality of radiographic images obtained by successive imaging using the grid; and
a suppression means that suppresses, based on the frequency characteristic, the periodic pattern in the one of the plurality of radiographic images and at least one radiographic image other than the one of the plurality of radiographic images.

9. The image processing apparatus, as defined in Claim 8, wherein the suppression means generates, based on the frequency characteristic, a filter for extracting a frequency component corresponding to the periodic pattern for each of the plurality of radiographic images, and obtains a plurality of radiographic images on which filtering processing has been performed by performing filtering processing on each of the plurality of radiographic images by using the filters, and suppresses the periodic pattern in the one of the plurality of radiographic images and at least one radiographic image other than the one of the plurality of radiographic images by subtracting the plurality of radiographic images on which filtering processing has been performed from the plurality of radiographic images.

10. The image processing apparatus, as defined in Claim 8 or 9, wherein successive imaging is tomosynthesis imaging.

11. An image processing method comprising:
obtaining a frequency characteristic of a periodic pattern caused by a grid for removing a scattered component of radiation by performing frequency analysis on one of a plurality of radiographic images obtained by successive imaging using the grid; and
suppressing, based on the frequency characteristic, the periodic pattern in the one of the plurality of radiographic images and at least one radiographic image other than the one of the plurality of radiographic images.
